# EUROPÄISCHE PATENTSCHRIFT

(11) **EP 0 723 544 B1**
(45) Veröffentlichungstag und Bekanntmachung des Hinweises auf die Patenterteilung: **18.04.2001**
(21) Anmeldenummer: 94930158.4
(22) Anmeldetag: 08.10.1994
(51) Int. Cl.: C07D 417/04, A61K 31/435

(54) **ALKOXYALKYLCARBAMATE VON IMIDAZO[1,2-a]PYRIDINEN**
ALKOXY ALKYL CARBAMATES OF IMIDAZO[1,2-a]PYRIDINES
CARBAMATES D'ALCOXYALKYLE D'IMIDAZO[1,2-a]PYRIDINES

(30) Priorität: 11.10.1993 CH 304793
(43) Veröffentlichungstag der Anmeldung: 31.07.1996
(73) Patentinhaber: Byk Gulden Lomberg Chemische Fabrik GmbH, 78467 Konstanz (DE)
(72) Erfinder: SENN-BILFINGER, Jörg, D-78464 Konstanz (DE); GRUNDLER, Gerhard, D-78464 Konstanz (DE); RIEDEL, Richard, D-88339 Bad Waldsee (DE); POSTIUS, Stefan, D-78467 Konstanz (DE); SIMON, Wolfgang-Alexander, D-78464 Konstanz (DE); RAINER, Georg, D-78464 Konstanz (DE)
(86) Internationale Anmeldenummer: EP9403326
(87) Internationale Veröffentlichungsnummer: WO9510518

(56) Entgegenhaltungen:
- EP-A- 0 033 094
- EP-A- 0 268 989
- EP-A- 0 308 917
- DATABASE WPI Week 9105, Derwent Publications Ltd., London, GB; AN 90-373004 & JP,A,2 270 873 (FUJISAWA PHARM. CO., LTD.) 5. November 1990

## Beschreibung

### Anwendungsgebiet der Erfindung

Die Erfindung betrifft neue Verbindungen, die in der pharmazeutischen Industrie als Wirkstoffe für die Herstellung von Arzneimitteln verwendet werden sollen.

### Bekannter technischer Hintergrund

In der europäischen Patentanmeldung EP-A-0 033 094 werden Imidazo[1,2-a]pyridine beschrieben, die in 8-Position einen Arylsubstituenten tragen, der bevorzugt ein Phenyl-, Thienyl-, Pyridyl- oder ein durch Chlor, Fluor, Methyl, tert.-Butyl, Trifluormethyl, Methoxy oder Cyan substituierter Phenylrest ist. Als besonders interessante Arylreste sind in der EP-A-0 033 094 die Reste Phenyl, o- oder p-Fluorphenyl, p-Chlorphenyl und 2,4,6-Trimethylphenyl genannt, wovon die Reste Phenyl, o- oder p-Fluorphenyl und 2,4,6-Trimethylphenyl besonders bevorzugt sind. - In den europäischen Patentanmeldungen EP-A-0 204 285, EP-A-0 228 006, EP-A-0 268 989 und EP-A-0 308 917 werden Imidazo[1,2-a]pyridine beschrieben, die in 3-Position einen ungesättigten aliphatischen Rest, insbesondere einen (substituierten) Alkinylrest tragen. - In der europäischen Patentanmeldung EP-A-0 266 890 werden Imidazo[1,2-a]pyridine beschrieben, die in 8-Position durch einen Alkenyl-, Alkyl- oder Cycloalkylalkylrest substituiert sind.

### Beschreibung der Erfindung

Es wurde nun gefunden, daß die nachfolgend näher beschriebenen Verbindungen, die sich von den Verbindungen des Standes der Technik insbesondere durch die Substitution in 3- oder in 8-Position unterscheiden, überraschende und besonders vorteilhafte Eigenschaften besitzen.

Gegenstand der Erfindungen sind Verbindungen der Formel I (siehe beigefügtes Formelblatt), worin
R1 1-4C-Alkyl,
R2 1-4C-Alkyl,
R3 1-4C-Alkoxy-2-4C-alkoxy,
R4 1-4C-Alkyl oder Hydroxymethyl und
A 0 (Sauerstoff) oder NH bedeutet,
und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und insbesondere der Methylrest.

1-4C-Alkoxy steht für ein Sauerstoffatom, an das einer der vorstehend genannten 1-4C-Alkylreste gebunden ist. Bevorzugt ist der Methoxyrest. 2-4C-Alkoxy steht für ein Sauerstoffatom, an das ein 2-4C-Alkylrest (ausgewählt aus den vorstehend genannten 1-4C-Alkylresten) gebunden ist. 1-4C-Alkoxy-2-4C-alkoxy steht für einen 2-4C-Alkoxyrest, an den ein 1-4C-Alkoxyrest gebunden ist. Bevorzugt ist der 2-Methoxyethoxyrest.

Als Salze kommen für Verbindungen der Formel I bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Als beispielhafte Verbindungen sind zu nennen die Verbindungen 8-{2-[(2-Methoxyethoxy)carbonylamino]-6-methylbenzyloxy}-2-methylimidazo-[1,2-a]pyridin-3-methanol, 8-{2-[(2-Methoxyethoxy)carbonylamino]-6-methylbenzylamino}-2-methylimidazo[1,2-a]pyridin-3-methanol und 8-{2-[(2-Methoxyethoxy)-carbonylamino]-6-methylbenzylamino}-2,3-dimethylimidazo[1,2-a]pyridin, und ihre Salze.

Hervorzuheben sind solche Verbindungen der Formel I, in denen R4 Hydroxymethyl und A 0 (Sauerstoff) bedeutet und R1, R2 und R3 die oben angegebenen Bedeutungen haben, und ihre Salze.

Weiterer Gegenstand der Erfindung ist ein Verfahren zur Herstellung der Verbindungen der Formel I und ihrer Salze. Das Verfahren ist dadurch gekennzeichnet, daß man
a) Verbindungen der Formel II (siehe das beiliegende Formelblatt), worin R1, R4 und A die oben angegebenen Bedeutungen haben, oder ihre Salze, mit Verbindungen der Formel III (siehe beiliegendes Formelblatt), worin R2 und R3 die oben angegebenen Bedeutungen haben und X eine geeignete reaktive Abgangsgruppe darstellt, oder ihren Salzen, umsetzt oder daß man
b) zur Herstellung von Verbindungen der Formel I, in denen R4 Hydroxymethyl bedeutet, Verbindungen der Formel IV (siehe beiliegendes Formel-blatt), worin R1, R2, R3 und A die oben angegebenen Bedeutungen haben, reduziert
und daß man gewünschtenfalls anschließend die nach a) oder b) erhaltenen Verbindungen I in ihre Salze überführt, oder daß man gewünschtenfalls anschließend aus erhaltenen Salzen der Verbindungen I die Verbindungen I freisetzt.

Die Umsetzung der Verbindungen II mit den Verbindungen III erfolgt auf eine dem Fachmann an sich vertraute Weise. Eine geeignete reaktive Abgangsgruppe ist beispielsweise ein Halogenatom (bevorzugt Chlor oder Brom) oder eine Methansulfonyloxygruppe. Die Umsetzung erfolgt vorteilhafterweise in Gegenwart einer Base (z.B. eines anorganischen Hydroxids, wie Natriumhydroxid, oder eines anorganischen Carbonates, wie Kaliumcarbonat, oder einer organischen Stickstoffbase, wie Triethylamin, Pyridin, Kollidin oder 4-Dimethylaminopyridin), wobei durch Zusatz von Katalysatoren, wie Alkalijodid oder Tetrabutylammoniumbromid, die Reaktionsführung begünstigt werden kann.

Die Reduktion der Verbindungen IV wird in einer dem Fachmann an sich gewohnten Weise vorgenommen. Sie erfolgt in inerten Lösungsmitteln, z.B. niederen aliphatischen Alkoholen, z.B. unter Verwendung geeigneter Hydride, wie beispielsweise Natriumborhydrid, gewünschtenfalls unter Zusatz von Wasser.

Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, einem niedermolekularen aliphatischen Alkohol (Ethanol, Isopropanol), einem Keton, wie Aceton, oder einem Ether, wie THF oder Diisopropylether, das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen II sind u.a. aus den europäischen Patentanmeldungen EP-A-0 290 003 und EP-A-0 299 470 bekannt. Die Ausgangsverbindungen III sind neu. Sie werden analog zu literaturbekannten Verfahren hergestellt, indem man in Verbindungen III mit X = OH die Hydroxygruppe in eine reaktive Abgangsgruppe umwandelt, z.B. in ein Halogenatom, durch Umsetzung mit einem Halogenierungsmittel, wie z.B. Thionylchlorid, Thionylbromid, Phosphortribromid oder Oxalylchlorid, oder in eine Methansulfonyloxygruppe, durch Umsetzung mit Methansulfonsäurechlorid, gewünschtenfalls in Gegenwart einer Base.

Die Verbindungen IV sind neu und ebenfalls Gegenstand der Erfindung. Sie werden hergestellt wie die Verbindungen der Formel I durch analoge Umsetzung von Verbindungen II mit R4 = CH0 mit Verbindungen III wie vorstehend beschrieben.

Die folgenden Beispiele dienen der näheren Erläuterung des Verfahrens zur Herstellung der Verbindungen I. Die Abkürzung RT steht für Raumtemperatur, h steht für Stunde(n).

### Beispiele

**End- und Zwischenprodukte**

### 1. 8-{2-[(2-Methoxyethoxy)carbonylamino]-6-methylbenzyloxy}-2-methylimidazo[1,2-a]pyridin-3-carboxaldehyd

Man rührt eine Mischung von 2,0 g (11,35 mmol) 8-Hydroxy-2-methylimidazo-[1,2-a]pyridin-3-carboxaldehyd, 1,2 g wasserfreiem Natriumcarbonat, 0,17 g (1,14 mmol) Natriumjodid und 3,3 g (12,8 mmol) [2-(Chlormethyl)-3-methylphenyl]carbamidsäure(2-methoxyethyl)ester in 30 ml Aceton 24 h bei RT und gießt auf 200 ml Eiswasser. Der Niederschlag wird abfiltriert, getrocknet und aus Toluol/Diisopropylether umkristallisiert. Man erhält 3,9 g (86,5 %) der Titelverbindung vom Schmp. 119-120°C.

### 2. 8-{2-[(2-Methoxyethoxy)carbonylamino]-6-methylbenzyloxy}-2-methylimidazo[1,2-a]pyridin-3-methanol

Man versetzt eine Suspension von 3,7 g (9,3 mmol) 8-{2-[(2-Methoxyethoxy)carbonylamino]-6-methylbenzyloxy}-2-methylimidazo[1,2-a]pyridin-3-carboxaldehyd in 40 ml Methanol bei RT mit 362 mg (9,3 mmol) 97 % Natriumborhydrid und rührt 75 Min. Man gibt auf Eis/Wasser, extrahiert mit Dichlormethan und konzentriert im Rotavapor. Das zurückbleibende Öl wird mit 5 ml Isopropylalkohol, 5 ml Toluol und Diisopropylether zur Kristallisation gebracht. Man erhält 2,7 g (72,7 %) der Titelverbindung vom Schmp. 121-123°C.

### 3. 8-{2-[(2-Methoxyethoxy)carbonylamino]-6-methylbenzylamino}-2-methyl-imidazo[1,2-a]pyridin-3-carboxaldehyd

Man rührt eine Mischung von 2,0 g (11,41 mmol) 8-Amino-2-methylimidazo-[1,2-a]pyridin-3-carboxaldehyd, 1,21 g (11,41 mmol) wasserfreiem Natriumcarbonat, 0,17 g (1,14 mmol) Natriumjodid und 3,5 g (13,6 mmol) [2-(Chlormethyl)-3-methylphenyl]carbamidsäure(2-methoxyethyl)ester in 30 ml Aceton 24 h bei RT und engt im Rotavapor ein. Man versetzt den Rückstand mit 100 ml Wasser und extrahiert mit Ethylacetat, trocknet die organische Phase mit Magnesiumsulfat und konzentriert im Vakuum. Der Rückstand wird aus Toluol umkristallisiert. Man erhält 3,31 g (73 %) der Titelverbindung vom Schmp. 153-155°C.

### 4. 8-{2-[(2-Methoxyethoxy)carbonylamino]-6-methylbenzylamino}-2-methyl-imidazo[1,2-a]pyridin-3-methanol

Man reduziert 2,8 g (7,06 mmol) 8-{2-[(2-Methoxyethoxy)carbonylamino]-6-methylbenzylamino}-2-methylimidazo[1,2-a]pyridin-3-carboxaldehyd mit Natriumborhydrid analog Beispiel 2, destilliert Methanol im Vakuum ab, versetzt mit Wasser und Ethylacetat und stellt mit Kaliumhydrogenphosphatlösung auf pH 9. Man extrahiert mehrmals mit Ethylacetat, trocknet, engt im Vakuum ein und kristallisiert aus Toluol/Diisopropylether um. Man erhält 2,28 g (81 %) der Titelverbindung vom Schmp. 138-140°C.

### 5. 8-{2-[(2-Methoxyethoxy)carbonylamino]-6-methylbenzylamino}-2,3-dimethylimidazo[1,2-a]pyridin - Isopropylalkohol (1/1)

Man rührt eine Mischung von 3,0 g (18,6 mmol) 8-Amino-2,3-dimethylimidazo[1,2-a]pyridin, 4,9 g (46,2 mmol) wasserfreiem Natriumcarbonat, 0,28 g (1,86 mmol) Natriumjodid und 5,8 g (22,5 mmol) [2-(Chlormethyl)-3-methylphenyl]carbamidsäure(2-methoxyethyl)ester in 30 ml Aceton 20 h bei RT. Man filtriert, engt im Vakuum ein, versetzt mit Wasser und Ethylacetat, stellt mit verdünnter Salzsäure auf pH 6 und extrahiert mit Ethylacetat. Die organische Lösung wird getrocknet und im Vakuum eingeengt. Der Rückstand wird mit 40 ml Aceton versetzt und mit einer Lösung von 1,2 g (10,3 mmol) Fumarsäure in 80 ml Aceton versetzt. Es erfolgt keine Kristallisation. So versetzt man die wieder eingeengte Lösung mit Toluol und Isopropylalkohol und fällt bei 0°C mit Diisopropylether 4,9 g eines Fumarats. Dieses versetzt man mit 50 ml Ethylacetat und 10 ml Wasser, stellt mit Natronlauge auf pH 9 und extrahiert die freie Base mit Ethylacetat. Nach dem Einengen im Vakuum löst man in Toluol/Isopropylalkohol und fällt bei 0°C mit Petrolether (Kp. 40°C). Man erhält 2,2 g (26,7 %) der Titelverbindung vom Schmp. 85-86°C.

### 6. 8-{2-[(2-Methoxyethoxy)carbonylaminol-6-methylbenzyloxy}-2-methylimidazo[1,2-a]pyridin-3-methanol

Man rührt eine Mischung von 178 mg (1,0 mmol) 8-Hydroxy-2-methylimidazo-[1,2-a]pyridin-3-methanol, 117 mg (1,1 mmol) wasserfreiem Natriumcarbonat, 15 mg (0,1 mmol) Natriumjodid und 283 mg (1,1 mmol) [2-(Chlormethyl)-3-methylphenyl]carbamidsäure(2-methoxyethyl)ester in 5 ml Aceton 48 h bei RT, arbeitet analog Beispiel 2 auf und chromatographiert mittels Ethylacetat/Isopropylalkohol (9:1). Man erhält 247 mg (62 %) der Titelverbindung.

### 7. 8-{2-[(2-Methoxyethoxy)carbonylaminol-6-methylbenzyloxy}-2,3-dimethyl-imidazo[1,2-a]pyridin

Analog Beispiel 5 werden 2,0 g (12,4 mmol) 8-Hydroxy-2,3-dimethylimidazo[1,2-a]pyridin, 3,6 g (13,9 mmol) [2-(Chlormethyl)-3-methylphenyl]carbamidsäure(2-methoxyethyl)ester, 0,18 g Natriumiodid und 1,3 g Natriumcarbonat in 30 ml Aceton umgesetzt. Man erhält 1,07 g (22,5 %) der Titelverbindung vom Schmp. 107-108°C.

### Ausgangsprodukte

Aa. [2-(Hydroxymethyl)-3-methylphenyl]carbamidsäure(2-methoxyethyl)ester
   Zu einer Lösung von 33 g (0,24 mol) 2-Amino-6-methylbenzylalkohol und 19,4 ml (0,24 mol) Pyridin in 600 ml Isopropylalkohol tropft man bei 10°C unter Rühren und Kühlen 33,2 g (0,24 mol) Chlorameisensäure(2-methoxyethyl)ester. Man rührt noch 2 h bei 0°C, versetzt mit Wasser und Isopropylacetat und extrahiert mehrmals mit Isopropylacetat. Die organische Phase wird mit Magnesiumsulfat getrocknet und bei 50°C im Rotavapor konzentriert. Der Rückstand wird auf einer Kieselgelsäule mittels Ethylacetat chromatographiert. Nach dem Einengen im Vakuum erhält man 36 g (68 %) der Titelverbindung als Öl.
Ab. [2-(Chlormethyl)-3-methylphenyl]carbamidsäure(2-methoxyethyl)ester
   Zu einer Lösung von 18,0 g (0,075 mol) der voranstehenden Verbindung in 80 ml Toluol tropft man unter Rühren und Kühlen 9,4 g (0,079 mol) Thionylchlorid bei 17-20°C zu und läßt über Nacht bei RT stehen. Man kühlt im Eisbad, reibt an und erhält 11,2 g (57,7 %) der Titelverbindung vom Schmp. 100-102°C. Durch Einengen der Mutterlauge und Kristallisieren aus Toluol/Petrolether (Kp. 40°C) erhält man eine zweite Fällung von 4,8 g (24,7 %) mit ähnlichem Schmelzpunkt.
B. 8-Hydroxy-2-methylimidazo[1,2-a]pyridin-3-carboxaldehyd
   4,77 g (0,02 mol) 8-Benzyloxy-2-methylimidazo[1,2-a]pyridin werden in einer Vilsmeier-Mischung aus 20 ml Dimethylformamid und 2,3 ml Phosphoroxychlorid 2,5 h bei 60°C gerührt und in üblicher Weise mit Eis/Wasser und Kaliumhydrogencarbonat aufgearbeitet. Man erhält 8-Benzyloxy-2-methylimidazo-[1,2-a]pyridin-3-carboxaldehyd vom Schmp. 105-106°C (aus Diisopropylether). Diese Verbindung wird analog Kaminski et al., J.Med.Chem.28,876(1985), Methode H, zur Titelverbindung vom Schmp. 251-252°C debenzyliert.

### Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgeprägte Magensäuresekretionshemmung und eine ausgezeichnete Magen- und Darmschutzwirkung bei Warmblütern auf. Hierbei zeichnen sich die erfindungsgemäßen Verbindungen neben einer guten Löslichkeit im wäßrigen Medium durch eine hohe Wirkungsselektivität, eine vergleichsweise lange Wirkungsdauer, eine gute enterale Wirksamkeit, das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen (z.B. Helicobacter pylori), Bakterientoxine, Medikamente (z.B. bestimmte Antiphlogistika und Antirheumatika), Chemikalien (z.B. Ethanol), Magensäure oder Streßsituationen verursacht werden können. Die erfindungsgemäßen Verbindungen besitzen hierbei auch eine Eigenwirkung gegen den Keim Helicobacter pylori.

In ihren ausgezeichneten Eigenschaften erweisen sich die erfindungsgemäßen Verbindungen an verschiedenen Modellen, in denen die antiulcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und/oder Prophylaxe von Erkrankungen des Magens und/oder Darms, aber auch zur Behandlung der Osteoporose verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfs- und/oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt und wobei durch die Wahl der Hilfs- und Trägerstoffe eine auf den Wirkstoff und/oder auf den gewünschten Wirkungseintritt genau angepaßte galenische Darreichungsform (z.B. eine Retardform oder eine magensaftresistente Form) erzielt werden kann.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Tablettenüberzugsmittel, Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise H₂-Blockern (z.B. Cimetidin, Ranitidih), H⁺/K⁺-ATPase-Hemmstoffen (z.B. Omeprazol, Pantoprazol), oder ferner mit sogenannten peripheren Anticholinergika (z.B. Pirenzepin, Telenzepin) sowie mit Gastrin-Antagonisten mit dem Ziel, die Hauptwirkung in additivem oder überadditivem Sinn zu verstärken und/oder die Nebenwirkungen zu eliminieren oder zu verringern, oder ferner die Kombination mit antibakteriell wirksamen Substanzen (wie z.B. Cephalosporinen, Tetracyclinen, Nalidixinsäure, Penicillinen oder auch Wismutsalzen) zur Bekämpfung von Helicobacter pylori.

### Pharmakologie

Die ausgezeichnete Magenschutzwirkung und die magensäuresekretionshemmende Wirkung der erfindungsgemäßen Verbindungen kann in Untersuchungen an tierexperimentellen Modellen nachgewiesen werden. Die in dem nachstehend aufgeführten Modell untersuchten erfindungsgemäßen Verbindungen sind mit Nummern versehen worden, die den Nummern dieser Verbindungen in den Beispielen entsprechen.

### Prüfung der sekretionshemmenden Wirkung am perfundierten Rattenmagen

In der folgenden Tabelle 1 ist der Einfluß der erfindungsgemäßen Verbindungen nach intravenöser Gabe auf die durch Pentagastrin stimulierte Säuresekretion des perfundierten Rattenmagens in vivo dargestellt.

**Tabelle 1**

| Nr. | Dosis (µmol/kg) i.v. | maximale Hemmung der Säureausscheidung während 3,5 h gegenüber Vorwert in % |
|---|---|---|
| 2 | 1 | 97 |
| 4 | 1 | 83 |
| 5 | 1 | 80 |
| 7 | 1 | 92 |

### Methodik

Narkotisierten Ratten (CD-Ratte, weiblich, 200-250 g; 1,5 g/kg i.m. Urethan) wurde nach Tracheotomie das Abdomen durch einen medianen Oberbauchschnitt eröffnet und ein PVC-Katheter transoral im Ösophagus sowie ein weiterer via Pylorus derart fixiert, daß die Schlauchenden eben noch in das Magenlumen hineinragten. Der aus dem Pylorus führende Katheter führte über eine seitliche Öffnung in der rechten Bauchwand nach außen.

Nach gründlicher Spülung (ca. 50-100 ml) wurde der Magen mit 37°C warmer physiologischer NaCl-Lösung kontinuierlich durchströmt (0,5 ml/min, pH 6,8-6,9; Braun-Unita I). In dem jeweils im 15 Min.-Abstand aufgefangenen (25 ml Meßzylinder) Effluat wurde der pH-Wert (pH-Meter 632, Glaselektrode EA 147; φ = 5 mm, Metrohm) sowie durch Titration mit einer frisch zubereiteten 0,01 N NaOH bis pH 7 (Dosimat 655 Metrohm) die sezernierte HCl bestimmt.

Die Stimulation der Magensekretion erfolgte durch Dauerinfusion von 1 µg/kg (= 1,65 ml/h) i.v. Pentagastrin (V. fem. sin.) ca. 30 Min. nach Operationsende (d.h. nach Bestimmung von 2 Vorfraktionen). Die zu prüfenden Substanzen wurden intravenös in 1 ml/kg Flüssigkeitsvolumen 60 Min. nach Beginn der Pentagastrin-Dauerinfusion verabreicht.

Die Körpertemperatur der Tiere wurde durch Infrarot-Bestrahlung und Heizkissen (automatische, stufenlose Regelung über rektalen Temperaturfühler) auf konstant 37,8 - 38°C gehalten.

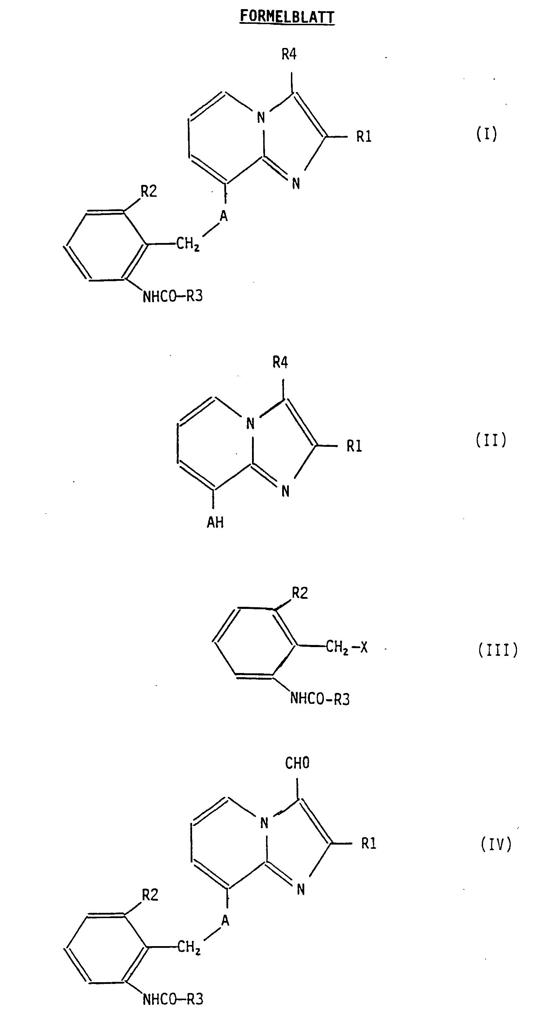

## Patentansprüche

1. Verbindungen der Formel I 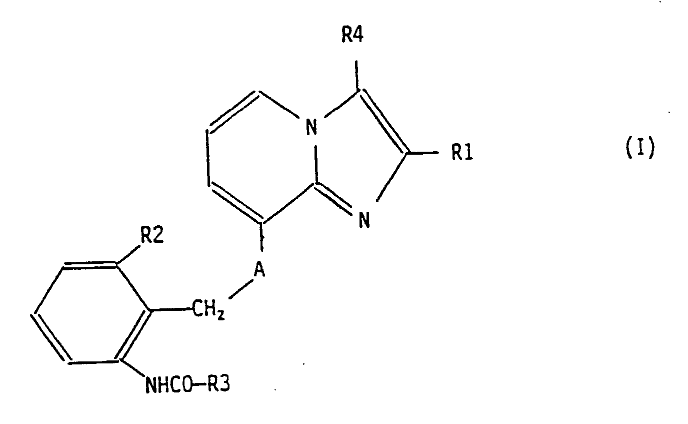 worin
R1 1-4C-Alkyl,
R2 1-4C-Alkyl,
R3 1-4C-Alkoxy-2-4C-alkoxy,
R4 1-4C-Alkyl oder Hydroxymethyl und
A 0 (Sauerstoff) oder NH bedeutet,
und ihre Salze.

2. Verbindungen der Formel I nach Anspruch 1, in denen R4 Hydroxymethyl und A 0 (Sauerstoff) bedeutet und R1, R2 und R3 die in Anspruch 1 angegebenen Bedeutungen haben, und ihre Salze.

3. 8-{2-[(2-Methoxyethoxy)carbonylamino]-6-methylbenzyloxy}-2-methylimidazo[1,2-a]pyridin-3-methanol und seine Salze.

4. 8-{2-[(2-Methoxyethoxy)carbonylamino]-6-methylbenzylamino}-2-methyl-imidazo[1,2-a]pyridin-3-methanol und seine Salze.

5. 8-{2-[(2-Methoxyethoxy)carbonylamino]-6-methylbenzylamino}-2,3-dimethylimidazo[1,2-a]pyridin und seine Salze.

6. Verfahren zur Herstellung der Verbindungen der Formel I nach Anspruch 1 und ihrer Salze, dadurch gekennzeichnet, daß man
a) Verbindungen der Formel II 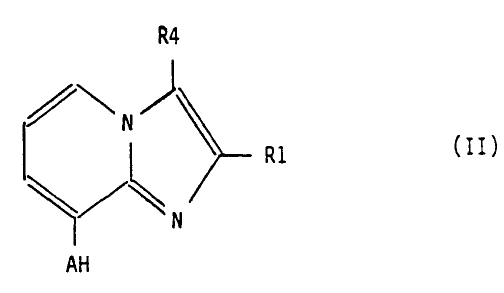 worin R1, R4 und A die in Anspruch 1 angegebenen Bedeutungen haben, oder ihre Salze, mit Verbindungen der Formel III 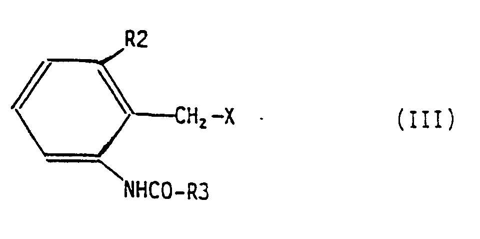 worin R2 und R3 die in Anspruch 1 angegebenen Bedeutungen haben und X eine geeignete reaktive Abgangsgruppe darstellt, oder ihren Salzen, umsetzt oder daß man
b) zur Herstellung von Verbindungen der Formel I, in denen R4 Hydroxymethyl bedeutet, Verbindungen der Formel IV 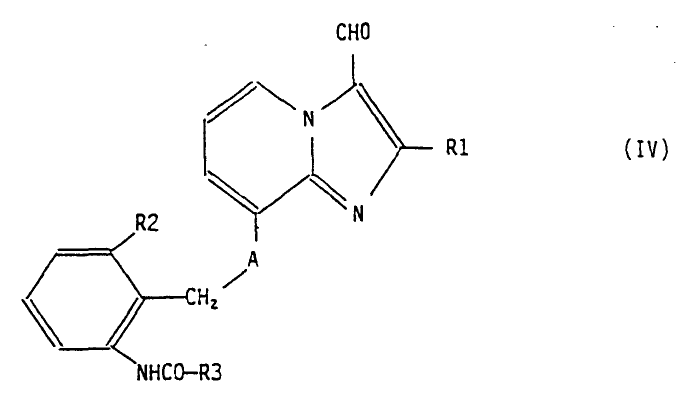 worin R1, R2, R3 und A die in Anspruch 1 angegebenen Bedeutungen haben, reduziert
und daß man gewünschtenfalls anschließend die nach a) oder b) erhaltenen Verbindungen I in ihre Salze überführt, oder daß man gewünschtenfalls anschließend aus erhaltenen Salzen der Verbindungen I die Verbindungen I freisetzt.

7. Arzneimittel enthaltend eine Verbindung nach Anspruch 1 und/oder ein pharmakologisch verträgliches Salz davon.

8. Verbindungen nach Anspruch 1 und ihre pharmakologisch verträglichen Salze zur Anwendung bei der Verhütung und Behandlung gastrointestinaler Krankheiten.

9. Verwendung von Verbindungen nach Anspruch 1 und ihren pharmakologisch verträglichen Salzen zur Herstellung von Arzneimitteln für die Verhütung und Behandlung gastrointestinaler Krankheiten.

## Claims

1. Compounds of the formula I 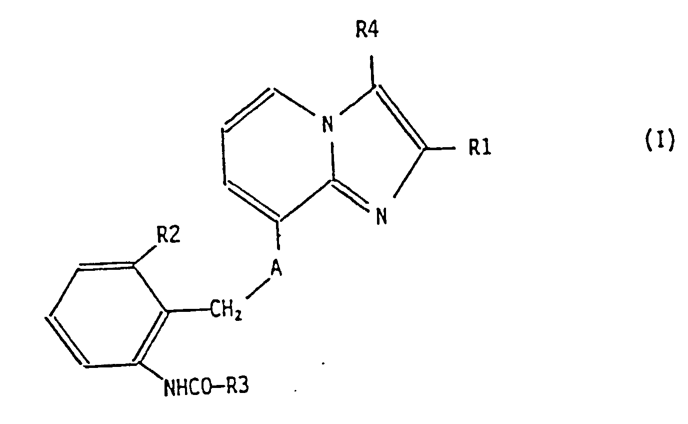 in which
R1 is 1-4C-alkyl,
R2 is 1-4C-alkyl,
R3 is 1-4C-alkoxy-2-4C-alkoxy,
R4 is 1-4C-alkyl or hydroxymethyl and
A is O (oxygen) or NH,
and their salts.

2. Compounds of the formula I according to Claim 1, in which R4 is hydroxymethyl and A is O (oxygen) and R1, R2 and R3 have the meanings indicated in Claim 1, and their salts.

3. 8-{2-[(2-Methoxyethoxy)carbonylamino]-6-methyl-benzyloxy}-2-methylimidazo[1,2-a]pyridine-3-methanol and its salts.

4. 8-{2-[(2-Methoxyethoxy) carbonylamino] -6-methyl-benzylamino}-2-methylimidazo[1,2-a]pyridine-3-methanol and its salts.

5. 8-{2-[(2-Methoxyethoxy) carbonylamino] -6-methyl-benzylamino}-2,3-dimethylimidazo[1,2-a]pyridine and its salts.

6. Process for the preparation of the compounds of the formula I according to Claim 1 and their salts, characterized in that
a) compounds of the formula II 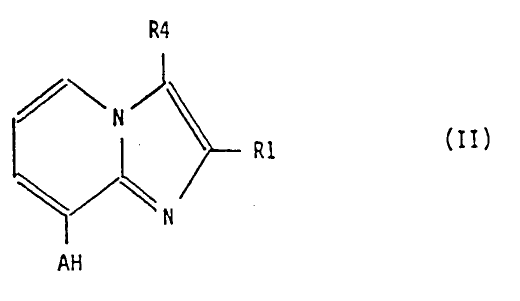 in which R1, R4 and A have the meanings indicated in Claim 1, or their salts, are reacted with compounds of the formula III 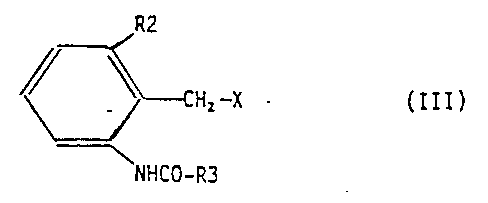 in which R2 and R3 have the meanings indicated in Claim 1 and X is a suitable reactive leaving group, or their salts, or in that
b) for the preparation of compounds of the formula I in which R4 is hydroxymethyl, compounds of the formula IV 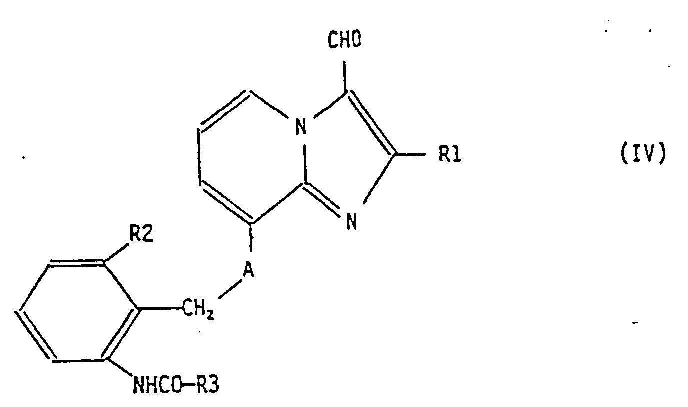 in which R1, R2, R3 and A have the meanings indicated in Claim 1, are reduced
and in that, if desired, the compounds I obtained according to a) or b) are then converted into their salts, or in that, if desired, the compounds I are then liberated from salts of the compounds I obtained.

7. Medicament comprising a compound according to Claim 1 and/or a pharmacologically tolerable salt thereof.

8. Compounds according to Claim 1 and their pharmacologically tolerable salts for use in the prevention and treatment of gastrointestinal diseases.

9. Use of compounds according to Claim 1 and their pharmacologically tolerable salts for the production of medicaments for the prevention and treatment of gastrointestinal diseases.

## Revendications

1. Composés de formule (I) 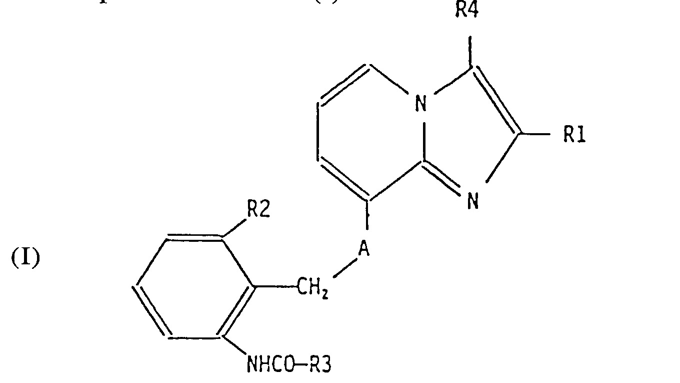 dans laquelle
R₁ représente un groupe alkyle en C₁₋₄,
R₂ représente un groupe alkyle en C₁₋₄,
R₃ représente un groupe (alcoxy en C₁₋₄)-(alcoxy en C₂₋₄)
R₄ représente un groupe alkyle en C₁₋₄ ou hydroxyméthyle et
A représente un atome d'oxygène ou un groupe NH,
ainsi que les sels de ces composés.

2. Composés de formule (I) selon la revendication 1, dans lesquels R₄ représente un groupe hydroxyméthyle et A un atome d'oxygène, et R₁, R₂ et R₃ ont la signification indiquée dans la revendication 1, et leurs sels.

3. 8-{2-[(2-méthoxyéthoxy)carbonylamino]-6-méthylbenzyloxy}-2-méthylimidazo[1,2-a]pyridine-3-méthanol et les sels de ce composé.

4. 8-{2-[(2-méthoxyéthoxy)carbonylamino]-6-méthylbenzyl-amino}-2-méthylimidazo[1,2-a]pyridine-3-méthanol et les sels de ce composé.

5. 8-{2-[(2-méthoxyéthoxy)carbonylamino]-6-méthylbenzyl-amino}-2,3-diméthylimidazo[1,2-a]pyridine-3-méthanol et les sels de ce composé.

6. Procédé de préparation de composés de formule (I) selon la revendication 1 et des sels de ces composés, caractérisé par le fait
a) que l'on fait réagir un composé de formule (II) 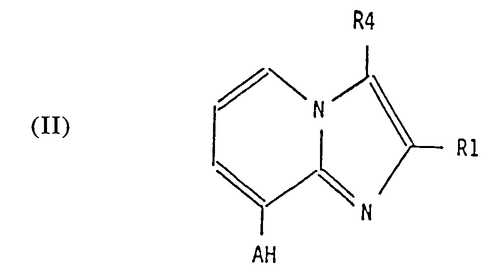 dans laquelle R₁, R₄ et A ont la signification indiquée dans la revendication 1, ou des sels d'un tel composé, avec un composé de formule (III) 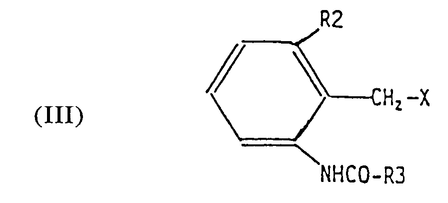 dans laquelle R₂ et R₃ ont la signification indiquée dans la revendication 1, et X représente un groupe partant réactif approprié, ou un sel d'un tel composé, ou
b) pour la préparation de composés de formule (I) dans laquelle R₄ représente un groupe hydroxyméthyle, que l'on soumet un composé de formule (IV) 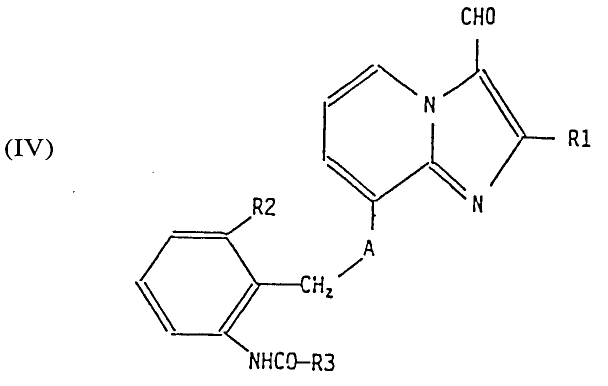 dans laquelle
R₁, R₂, R₃ et A ont la signification indiquée dans la revendication 1, à une réduction
et que l'on convertit éventuellement ensuite les composés obtenus dans l'étape a) ou b) en leurs sels, ou que l'on convertit éventuellement les sels de composés de formule (I) obtenus en les composés libres correspondants.

7. Médicament contenant un composé selon la revendication 1 et/ou un sel pharmacologiquement acceptable de celui-ci.

8. Composé selon la revendication 1 et leurs sels pharmacologiquement acceptables pour l'utilisation dans la prévention et le traitement d'affections gastrointestinales.

9. Utilisation de composés selon la revendication 1 et leur sels pharmacologiquement acceptables pour la fabrication de médicaments pour la prévention et le traitement d'affections gastrointestinales.
